(19) Europäisches Patentamt
European Patent Office
Office européen des brevets

(11) **EP 4 303 879 A1**

(12) **EUROPEAN PATENT APPLICATION**

(43) Date of publication:
**10.01.2024   Bulletin 2024/02**

(21) Application number: **22206560.9**

(22) Date of filing: **10.11.2022**

(51) International Patent Classification (IPC):
**G16C 99/00** (2019.01)

(52) Cooperative Patent Classification (CPC):
**G16C 99/00;** Y02W 10/10

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.07.2022   CN 202210794615**

(71) Applicant: **China Three Gorges Co., Ltd.**
**Wuhan Hubei 430010 (CN)**

(72) Inventors:
• **LIU, Xiaoting**
**Wuhan, 430010 (CN)**
• **DAI, Huichao**
**Wuhan, 430010 (CN)**

• **WEI, Song**
**Wuhan, 430010 (CN)**
• **LIU, Zhiwu**
**Wuhan, 430010 (CN)**
• **JIANG, Dingguo**
**Wuhan, 430010 (CN)**
• **ZHAO, Hanqing**
**Wuhan, 430010 (CN)**
• **ZHAI, Ran**
**Wuhan, 430010 (CN)**
• **ZHAI, Yanwei**
**Wuhan, 430010 (CN)**
• **ZHANG, Chengxiao**
**Wuhan, 430010 (CN)**

(74) Representative: **J A Kemp LLP**
**80 Turnmill Street**
**London EC1M 5QU (GB)**

(54) **METHOD AND APPARATUS FOR CONSTRUCTING TRANSPORT MODEL FOR DISSOLVED ORGANIC MATTERS IN RIVER, AND PREDICTING METHOD AND APPARATUS**

(57) The present invention discloses a method and apparatus for constructing a transport model for dissolved organic matters in a river, and a predicting method and apparatus. The method for constructing a transport model for dissolved organic matters in a river includes: acquiring first environmental parameters and first dissolved organic matter concentrations in a target river in a base flow stage and an incoming flow stage; determining a first dissolved organic matter transport mechanism in the target river according to the first environmental parameters and the first dissolved organic matter concentrations; and constructing a dissolved organic matter transport model, for dissolved organic matters in the target river in the base flow stage and the incoming flow stage, based on the first dissolved organic matter transport mechanism. The dissolved organic matter transport model constructed in the present invention can be used, for the dissolved organic matters in the river under a dynamic condition of a sudden change in an incoming flow, to assist in assessing the influence of incoming water, such as rainfall or flood discharge, on the distribution of organic matters in aquatic habitats in the river.

Acquiring first environmental parameters and first dissolved organic matter concentrations in a target river in a base flow stage and an incoming flow stage — 110

Determining a first dissolved organic matter transport mechanism in the target river according to the first environmental parameters and the first dissolved organic matter concentrations — 120

Constructing a dissolved organic matter transport model, for dissolved organic matters in the target river in the base flow stage and the incoming flow stage, based on the first dissolved organic matter transport mechanism — 130

Fig. 1

**Description**

**TECHNICAL FIELD**

Embodiments of the present invention relate to the technical field of engineering simulation and numerical simulation, and in particular to a method and apparatus for constructing a transport model for dissolved organic matters in a river, and a predicting method and apparatus.

**BACKGROUND**

A river in a natural state has a very low content of dissolved organic matters, which however, play a very important role in the aqueous environment, have strong reaction and migration activities, and have an important influence on the morphotoxicity and bioavailability of trace metal ions and organic pollutants in the water body and their migration and transformation. The dissolved organic matters are the most major part of organic components in the water body, also a common indicator for measuring the water quality, and anZ important basis for water quality monitoring and management. The dissolved organic matters, which are reactive solutes, have complex composition, and their migration process involves many complex biological, physical, chemical and other effects. It is difficult to precisely characterize their complex migration behaviors by a classical convective-diffusive equation and a traditional empirical formula. Especially in some topographically complex river channels, a migration process of the dissolved organic matters does not conform to Gaussian distribution, but has a serious tailing phenomenon.

Truncated fractional order derivatives are able to characterize historical memory of a particle movement process, and are therefore widely used. However, a dissolved organic matter transport process is influenced by the external environment. Especially in an upstream incoming flow process, a rainfall and other incoming flow changes have great influences on dissolved organic matter distribution. It is difficult to characterize the dissolved organic matter distribution in this process by an existing truncated fractional order derivative model of a single stage.

**SUMMARY**

To solve the problems in the prior art, the present application provides a method and apparatus for constructing a transport model for dissolved organic matters in a river, and a predicting method and apparatus.

In a first aspect, the present application provides a method for constructing a transport model for dissolved organic matters in a river, the method including:

acquiring first environmental parameters and first dissolved organic matter concentrations in a target river in a base flow stage and an incoming flow stage;

determining a first dissolved organic matter transport mechanism in the target river according to the first environmental parameters and the first dissolved organic matter concentrations; and

constructing a dissolved organic matter transport model, for dissolved organic matters in the target river in the base flow stage and the incoming flow stage, based on the first dissolved organic matter transport mechanism.

In a possible implementation, the first dissolved organic matter transport mechanism includes:
a physical adsorption ratio biodegradation process of the dissolved organic matters in the target river, and influences, by biochemical and physical effects, on a tailing degree of the dissolved organic matters.

In a possible implementation, the dissolved organic matter transport model is:

$$\begin{cases} \dfrac{\partial C(x,t)}{\partial t} + \beta_0 \, {}_0^{TC}D_t^{\alpha_0,\lambda_0} C(x,t) - \left(\beta_0 \lambda_0^{\alpha_0} - R_0\right) C(x,t) \\[4mm] = -v_0 \dfrac{\partial C(x,t)}{\partial x} + D_0 \dfrac{\partial^2 C(x,t)}{\partial x^2}, \; t \in \left[0, T_1\right] \\[4mm] \dfrac{\partial C(x,t)}{\partial t} + \beta_1 \, {}_{T_1}^{TC}D_t^{\alpha_1,\lambda_1} C(x,t) - \left(\beta_1 \lambda_1^{\alpha_1} - R_1\right) C(x,t) \\[4mm] = -v_1 \dfrac{\partial C(x,t)}{\partial x} + D_1 \dfrac{\partial^2 C(x,t)}{\partial x^2}, \; t \in \left[T_1, T_2\right] \\[4mm] C(x,0) = \begin{cases} A, & x = 0 \\ 0, & else \end{cases}, \; C\left(x, T_1^+\right) = C\left(x, T_1^-\right) \\[4mm] \dfrac{\partial C(x,t)}{\partial x}\Big|_{\partial\Omega} = 0 \end{cases}$$

where $x$, $t$ represents space and time, respectively, $C$ is a concentration of the dissolved organic matters in the target river, $T_I$ is upstream incoming flow start time in the target river, $T_2$ represents an incoming flow end time, $\beta$ represents a fractional order capacity coefficient, $\lambda$ represents a truncation coefficient, $\alpha$ represents a fractional order number, $R$ represents a reaction coefficient, $v$ and $D$ are a flow parameter and a diffusion parameter, respectively, $A$ represents an initial concentration of the dissolved organic matters, $\partial\Omega$ represents derivative calculation along a boundary, ${}_0^{TC}D_t^{\alpha_0,\lambda_0}$ and ${}_{T_1}^{TC}D_t^{\alpha_1,\lambda_1}$ are truncated fractional order derivatives, and the subscripts 0 and 1 of $\alpha$, $\beta$, $\lambda$, $R$, $v$ and $D$ represent time periods corresponding to the parameters, respectively, the parameters in the base flow stage being denoted with the subscript 0, and the parameters in the incoming flow stage being denoted with the subscript 1.

In a second aspect, the present application provides a method for predicting a transport process of dissolved organic matters in a river, the method including:

acquiring second environmental parameters and second dissolved organic matter concentrations in a target river in a base flow stage and an incoming flow stage;
determining a second dissolved organic matter transport mechanism in the target river according to the second environmental parameters and the second dissolved organic matter concentrations;
calibrating the parameters of the dissolved organic matter transport model constructed by the method in any embodiment in the first aspect according to the second dissolved organic matter concentrations and the second dissolved organic matter transport mechanism;
determining dissolved organic matter distribution based on the dissolved organic matter transport model and the calibrated model parameters; and
predicting a dissolved organic matter transport process in the incoming flow stage in the target river based on the dissolved organic matter distribution.

In a possible implementation, acquiring dissolved organic matter distribution based on the dissolved organic matter transport model and the calibrated model parameters includes:
acquiring dissolved organic matter distribution by solving the dissolved organic matter transport model using an implicit finite difference method, based on the dissolved organic matter transport model and the calibrated model parameters.
In a possible implementation, solving the dissolved organic matter transport model using the implicit finite difference method is specifically as follows:

when $t \in [0, T_1]$, the number of points distributed in time is m, a time step is $\tau = T_1/m$, and a spatial step is $h$, $t_{k+1}$

represents ($k$+1)th time, and $x_l$ represents an $l$th spatial position;

when $k$=0:

$$\left( \tau^{-1} + \beta_0 \frac{\tau^{-\alpha_0}}{\Gamma\left(2-\alpha_0\right)} - \frac{2D_0}{h^2} - \beta_0 \lambda_0^{\alpha_0} + R_0 \right) C\left(x_l, t_1\right)$$

$$+ \left( \frac{v_0}{h} + \frac{D_0}{h^2} \right) C\left(x_{l+1}, t_1\right)$$

$$+ \left( -\frac{v_0}{h} + \frac{D_0}{h^2} \right) C\left(x_{l-1}, t_1\right)$$

$$= \tau^{-1} C\left(x_l, 0\right) - \left( \beta_0 \frac{e^{-\lambda_0 t_1} \tau^{-\alpha_0}}{\Gamma\left(2-\alpha_0\right)} \right) C\left(x_l, 0\right)$$

when $k$=1:

$$\left( \tau^{-1} + \beta_0 \frac{\tau^{-\alpha_0}}{\Gamma\left(2-\alpha_0\right)} - \frac{2D_0}{h^2} - \beta_0 \lambda_0^{\alpha_0} + R_0 \right) C\left(x_l, t_2\right)$$

$$+ \left( \frac{v_0}{h} + \frac{D_0}{h^2} \right) C\left(x_{l+1}, t_2\right)$$

$$+ \left( -\frac{v_0}{h} + \frac{D_0}{h^2} \right) C\left(x_{l-1}, t_2\right)$$

$$= \tau^{-1} C\left(x_l, t_1\right) - \left( \beta_0 \frac{e^{-\lambda_0 t_2} \tau^{-\alpha_0}}{\Gamma\left(2-\alpha_0\right)} \right) \left[ \begin{array}{c} e^{\lambda_0 t_1} C\left(x_l, t_1\right)\left(2^{1-\alpha_0} - 2\right) \\ -C\left(x_l, 0\right)\left(2^{1-\alpha_0} - 1\right) \end{array} \right]$$

when k> 1:

$$\left( \tau^{-1} + \beta_0 \frac{\tau^{-\alpha_0}}{\Gamma\left(2-\alpha_0\right)} - \frac{2D_0}{h^2} - \beta_0 \lambda_0^{\alpha_0} + R_0 \right) C\left(x_l, t_{k+1}\right)$$

$$+ \left( \frac{v_0}{h} + \frac{D_0}{h^2} \right) C\left(x_{l+1}, t_{k+1}\right)$$

$$+ \left( -\frac{v_0}{h} + \frac{D_0}{h^2} \right) C\left(x_{l-1}, t_{k+1}\right)$$

$$= \tau^{-1} C\left(x_l, t_k\right) - \beta_0 \frac{e^{-\lambda_0 t_{k+1}} \tau^{-\alpha_0}}{\Gamma\left(2-\alpha_0\right)} e^{\lambda_0 t_k} C\left(x_l, t_k\right) \left(2^{1-\alpha_0} - 2\right)$$

$$- \beta_0 \frac{e^{-\lambda_0 t_{k+1}} \tau^{-\alpha_0}}{\Gamma\left(2-\alpha_0\right)} \sum_{j=1}^{k-1} e^{-\lambda_0 t_{k-j}} C\left(x_l, t_{k-j}\right) \left[ (j+2)^{1-\alpha_0} - 2(j+1)^{1-\alpha_0} + j^{1-\alpha_0} \right]$$

$$+ \beta_0 \frac{e^{-\lambda_0 t_{k+1}} \tau^{-\alpha_0}}{\Gamma\left(2-\alpha_0\right)} C\left(x_l, 0\right) \left[ j_{k+1}^{1-\alpha_0} - j_k^{1-\alpha_0} \right]$$

when $t \in [T_1, T_2]$, the number of points distributed in time is $m_1$, a time step is $\tau_1 = (T_2 - T_1)/m_1$, and a spatial step is still $h$, $t_{k+1}$ represents (k+1)th time, and $x_l$ represents an /th spatial position;

when $k-m=0$:

$$\left( \tau_1^{-1} + \beta_1 \frac{\tau_1^{-\alpha_1}}{\Gamma\left(2-\alpha_1\right)} - \frac{2D_1}{h^2} - \beta_1 \lambda_1^{\alpha_1} + R_1 \right) C\left(x_l, t_{k+1}\right)$$

$$+ \left( \frac{v_1}{h} + \frac{D_1}{h^2} \right) C\left(x_{l+1}, t_{k+1}\right)$$

$$+ \left( -\frac{v_1}{h} + \frac{D_1}{h^2} \right) C\left(x_{l-1}, t_{k+1}\right)$$

$$= \tau_1^{-1} C\left(x_l, t_k\right) - \left( \beta_1 \frac{e^{-\lambda_1\left(t_{k+1}-T_1\right)} \tau_1^{-\alpha_1}}{\Gamma\left(2-\alpha_1\right)} \right) C\left(x_l, T_1\right)$$

when $k-m=1$:

$$\left( \tau_1^{-1} + \beta_1 \frac{\tau_1^{-\alpha_1}}{\Gamma(2-\alpha_1)} - \frac{2D_1}{h^2} - \beta_1 \lambda_1^{\alpha_1} + R_1 \right) C\left(x_l, t_{k+1}\right)$$

$$+ \left( \frac{v_1}{h} + \frac{D_1}{h^2} \right) C\left(x_{l+1}, t_{k+1}\right)$$

$$+ \left( -\frac{v_1}{h} + \frac{D_1}{h^2} \right) C\left(x_{l-1}, t_{k+1}\right) = \tau_1^{-1} C\left(x_l, t_k\right)$$

$$- \left( \beta_1 \frac{e^{-\lambda_1(t_{k+1}-T_1)} \tau_1^{-\alpha_1}}{\Gamma(2-\alpha_1)} \right) \left[ \begin{array}{l} e^{\lambda_1(t_k-T_1)} C\left(x_l, t_k\right)\left(2^{1-\alpha_1} - 2\right) \\ - C\left(x_l, T_1\right)\left(2^{1-\alpha_1} - 1\right) \end{array} \right]$$

when $k-m \geq 1$:

$$\left( \tau_1^{-1} + \beta_1 \frac{\tau_1^{-\alpha_1}}{\Gamma(2-\alpha_1)} - \frac{2D_1}{h^2} - \beta_1 \lambda_1^{\alpha_1} + R_1 \right) C\left(x_l, t_{k+1}\right)$$

$$+ \left( \frac{v_1}{h} + \frac{D_1}{h^2} \right) C\left(x_{l+1}, t_{k+1}\right)$$

$$+ \left( -\frac{v_1}{h} + \frac{D_1}{h^2} \right) C\left(x_{l-1}, t_{k+1}\right)$$

$$= \tau_1^{-1} C\left(x_l, t_k\right) - \beta_1 \frac{e^{-\lambda_1(t_{k+1}-T_1)} \tau_1^{-\alpha_1}}{\Gamma(2-\alpha_1)} e^{\lambda_1(t_{k+1}-T_1)} C\left(x_l, t_k\right)\left(2^{1-\alpha_1} - 2\right)$$

$$- \beta_1 \frac{e^{-\lambda_1(t_{k+1}-T_1)} \tau_1^{-\alpha_1}}{\Gamma(2-\alpha_1)} \sum_{j=1}^{k-m-1} e^{-\lambda_1(t_{k-j}-T_1)} C\left(x_l, t_{k-j}\right)\left[ (j+2)^{1-\alpha_1} - 2(j+1)^{1-\alpha_1} + j^{1-\alpha_1} \right]$$

$$+ \beta_1 \frac{e^{-\lambda_1(t_{k+1}-T_1)} \tau_1^{-\alpha_1}}{\Gamma(2-\alpha_1)} C\left(x_l, T_1\right)\left[ j_{k+1}^{1-\alpha_1} - j_k^{1-\alpha_1} \right]$$

where *x, t* represent space and time, respectively, *C* is a concentration of the dissolved organic matters in the target river, $T_l$ is upstream incoming flow start time in the target river, $T_2$ represents incoming flow end time, $\beta$ represents a fractional order capacity coefficient, $\lambda$ represents a truncation coefficient, $\alpha$ represents a fractional order number, *R* represents a reaction coefficient, *v* and *D* are a flow parameter and a diffusion parameter, respectively, *A* represents an initial concentration of the dissolved organic matters, $\partial\Omega$ represents derivative calculation along a boundary,

$^{TC}_{0}D_t^{\alpha_0,\lambda_0}$ and $^{TC}_{T_1}D_t^{\alpha_1,\lambda_1}$ are truncated fractional order derivatives, the subscripts 0 and 1 of $\alpha$, $\beta$, A, $R$, v and D represent time periods corresponding to the parameters, respectively, the parameters in the base flow stage being denoted with the subscript 0, and the parameters in the incoming flow stage being denoted with the subscript 1, $\Gamma$ () is a single-parameter Gamma function, $t_1$ is initial time of a fractional order derivative, $t_2$ is end time of the fractional order derivative, and $e$ is a natural constant.

In a third aspect, the present invention provides an apparatus for constructing a transport model for dissolved organic matters in a river, the apparatus including:

a first acquisition module configured to acquire first environmental parameters and first dissolved organic matter concentrations in a target river in a base flow stage and an incoming flow stage;
a first transport mechanism module configured to determine a first dissolved organic matter transport mechanism in the target river according to the first environmental parameters and the first dissolved organic matter concentrations; and
a construction module configured to construct a dissolved organic matter transport model, for dissolved organic matters in the target river in the base flow stage and the incoming flow stage, based on the dissolved organic matter transport mechanism.

In a fourth aspect, the present invention provides an apparatus for predicting a transport process of dissolved organic matters in a river, the apparatus including:

a second acquisition module configured to acquire second environmental parameters and second dissolved organic matter concentrations in a target river in a base flow stage and an incoming flow stage;

a second transport mechanism module configured to determine a second dissolved organic matter transport mechanism in the target river according to the second environmental parameters and the second dissolved organic matter concentrations;

a first processing module configured to calibrate the parameters of the dissolved organic matter transport model constructed by the apparatus in the third aspect according to the second dissolved organic matter concentrations and the second dissolved organic matter transport mechanism;

a second processing module configured to determine dissolved organic matter distribution based on the dissolved organic matter transport model and the calibrated model parameters; and

a prediction module configured to predict a dissolved organic matter transport process in the incoming flow stage in the target river based on the dissolved organic matter distribution.

In a fifth aspect, the present application provides an electronic device, including a processor, a communication interface, a memory and a communication bus, wherein the processor, the communication interface and the memory communicate with each other via the communication bus;

the memory is configured to store a computer program; and

the processor is configured to implement the steps of the method for constructing a transport model for dissolved organic matters in a river in any embodiment in the first aspect when executing the program stored in the memory;

or the processor is configured to implement the steps of the method for predicting a transport process of dissolved organic matters in a river in any embodiment in the second aspect when executing the program stored in the memory.

In a sixth aspect, the present application provides a computer readable storage medium, with a computer program stored therein, wherein the computer program, when executed by a processor, implements the steps of the method for constructing a transport model for dissolved organic matters in a river in any embodiment in the first aspect; or, the computer program, when executed by a processor, implements the steps of the method for predicting a transport process of dissolved organic matters in a river in any embodiment in the second aspect.
Compared with the prior art, the above-mentioned technical solutions provided in embodiments of the present application have the following advantages:
according to the method for constructing a transport model for dissolved organic matters in a river provided in embodiments of the present application, the first environmental parameters and the first dissolved organic matter concentrations in the target river in the base flow stage and the incoming flow stage are acquired. The first dissolved organic matter transport mechanism in the target river is determined according to the first environmental parameters and the first dissolved organic matter concentrations, and the dissolved organic matter transport model is constructed, for dissolved organic matters in the target river in the base flow stage and the incoming flow stage, based on the first dissolved organic matter transport mechanism. The dissolved organic matter transport model constructed in the present invention can be used, for the dissolved organic matters in the river under a dynamic condition of a sudden change in an incoming flow, to assist in assessing the influence of the incoming flow, such as rainfall or flood discharge, on the distribution of dissolved organic matters in aquatic habitats in the river.

**BRIEF DESCRIPTION OF THE DRAWINGS**

Fig. 1 is a schematic flow diagram of a method for constructing a transport model for dissolved organic matters in a river provided in embodiments of the present invention;

Fig. 2 is a schematic flow diagram of a method for predicting a transport process of dissolved organic matters in a river provided in the present invention;

Fig. 3 shows simulation results of dissolved organic matters in national Mayfield Creek;

Fig. 4 is a schematic structural diagram of an apparatus for constructing a transport model for dissolved organic matters in a river provided in embodiments of the present invention;

Fig. 5 is a schematic structural diagram of an apparatus for predicting a transport process of dissolved organic matters in a river provided in embodiments of the present invention; and

Fig. 6 is a schematic structural diagram of an electronic device provided in embodiments of the present invention.

**DETAILED DESCRIPTION**

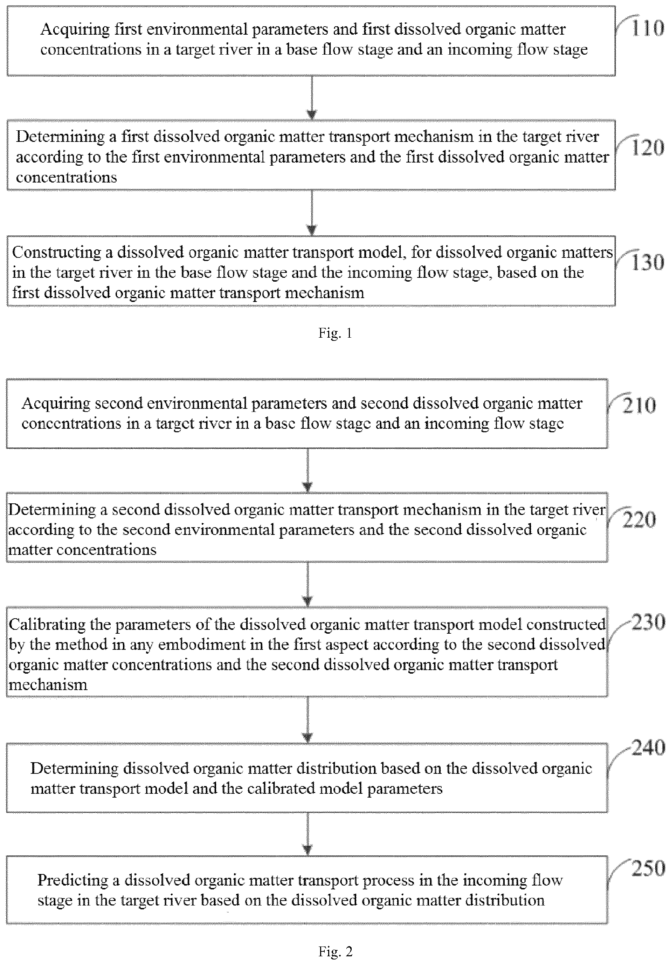

To make the objects, technical solutions and advantages of the embodiments of the present invention clearer, the technical solutions in the embodiments of the present invention will be described below clearly and completely in conjunction with the drawings in the embodiments of the present invention. Obviously, the embodiments described are part of, but not all of, the embodiments of the present invention. All other embodiments obtained by those of ordinary skill in the art without creative work, based on the embodiments in the present invention, fall into the protection scope of the present invention.
It should be noted that relational terms such as "first" and "second" herein are only used to distinguish one entity or operation from another entity or operation, and do not necessarily require or imply there is any such actual relationship or order between the entities or operations. Moreover, the terms "comprise", "include" or any other variations thereof are intended to encompass non-exclusive inclusion, such that a process, method, item or device that includes a series of elements includes not only the elements, but also other elements not explicitly listed, or elements inherent to this process, method, item or device. Without more restrictions, elements defined by the statement "comprising a..." do not exclude the presence of other identical elements in the process, method, item or device comprising the elements.
To facilitate understanding of the embodiments of the present invention, specific embodiments will be further explained in conjunction with the drawings, and the embodiments do not limit the embodiments of the present invention.
In view of the technical problems mentioned in the background, for dissolved organic matters in a river under a dynamic condition of a sudden change in an incoming flow such as rainfall or aerial discharge, a transport model that can simulate a transport process of the dissolved organic matters in the river is proposed to assist in assessing the influence of rainfall on the distribution of dissolved organic matters in aquatic habitats in the river. Specifically, embodiments of the present application provide a method for constructing a transport model for dissolved organic matters in a river. For a specific implementing process, see a schematic flow diagram of a method shown in Fig. 1. Fig 1 is a schematic flow diagram of a method for constructing a transport model for dissolved organic matters in a river, the method including steps as follows:
step 110: first environmental parameters and first dissolved organic matter concentrations in a target river in a base flow stage and an incoming flow stage are acquired.

Constructing a transport model for dissolved organic matters first needs to acquire experimental data for a certain time period, which is called a target stage in the present invention, i.e., in the target stage, experimental data are acquired by conducting dissolved organic matter tracer experiments in the target river in the base flow stage and the incoming flow stage, respectively, the experimental data including the first environmental parameters and the first dissolved organic matter concentrations in the target river. In an example, due to the difficulty of capturing experimental conditions for field rainfalls, dam aerial discharge here approximately represents an incoming flow after rainfall here. For a natural river with a dam upstream, in a base flow stage and under certain aerial discharge (also called incoming flow) conditions, respectively, a low-velocity region, a deep-water region, and a riverbed structure of the river are roughly determined by collecting historical data or performing field measurements, and experimental data for the target stage are acquired, which are specifically environment-related parameters, including a river base flow, dam aerial discharge, and environmental parameters of the river, such as a water level, pH, a flow rate, temperature etc. Fallen leaves are collected and soaked in river water to obtain leachate, or a colored soluble organic matter such as humic acid or fulvic acid is directly used as a tracer. Before an experiment, detection points are set at a first distance and at a second distance below the dam, respectively, e.g. monitoring stations are set at 50m and at 150m below the dam, respectively, and a concentration of the tracer organic matter in the river is monitored continuously. The tracer is placed 50m below the dam under base flow conditions. Depending on water flow conditions, when the tracer concentration is monitored at the 150m monitoring point, the dam discharges water at a fixed flow rate, which reflects a flow rate after upstream rainfall converges, and measured tracer organic matter concentrations are continuously collected and sorted to determine dissolved organic matter concentrations.

Step 120: a first dissolved organic matter transport mechanism in the target river is determined according to the first environmental parameters and the first dissolved organic matter concentrations.

According to the experimental data acquired in the above step, the first dissolved organic matter transport mechanism in the target river is analyzed, including a physical adsorption ratio biodegradation process of the dissolved organic matters in the target river, and influences, by biochemical and physical effects, on a tailing degree of the dissolved organic matters.

Specifically, according to the acquired experimental data, the physical adsorption ratio biodegradation process of the dissolved organic matters in the river, and the influences, by biochemical and physical effects, on the tailing degree of the dissolved organic matters are analyzed. Biodegradation and chemisorption weaken the tailing phenomenon. Physical effects (e.g., physical adsorption and desorption processes, and slow material exchange processes of dissolved organic matters in immobile regions, etc.) enhance the degree of tailing of a soluble organic matter breakthrough curve.

Step 130: a dissolved organic matter transport model is constructed, for dissolved organic matters in the target river in the base flow stage and the incoming flow stage, based on the first dissolved organic matter transport mechanism.

Specifically, according to the analysis in step 120, the transport model for dissolved organic matters in the river is established for two stages before and after rainfall. The dissolved organic matter transport model functions to reflect a transport process of organic matters containing a reaction item. The reaction item as used here is the organic matter tracer that can participate in a chemical reaction in step 1. In an example, the dissolved organic matter transport model is a truncated fractional order derivative model with dual stress sections, specifically a truncated fractional order convective-diffusive equation with dual stress sections:

$$
\begin{cases}
\dfrac{\partial C(x,t)}{\partial t} + \beta_0 \, {}^{TC}_{0}D_t^{\alpha_0,\lambda_0} C(x,t) - \left( \beta_0 \lambda_0^{\alpha_0} - R_0 \right) C(x,t) \\[2mm]
= -v_0 \dfrac{\partial C(x,t)}{\partial x} + D_0 \dfrac{\partial^2 C(x,t)}{\partial x^2}, \; t \in \left[0, T_1\right] \\[4mm]
\dfrac{\partial C(x,t)}{\partial t} + \beta_1 \, {}^{TC}_{T_1}D_t^{\alpha_1,\lambda_1} C(x,t) - \left( \beta_1 \lambda_1^{\alpha_1} - R_1 \right) C(x,t) \\[2mm]
= -v_1 \dfrac{\partial C(x,t)}{\partial x} + D_1 \dfrac{\partial^2 C(x,t)}{\partial x^2}, \; t \in \left[T_1, T_2\right] \\[4mm]
C(x,0) = \begin{cases} A, \; x = 0 \\ 0, \; else \end{cases}, \; C\left(x, T_1^+\right) = C\left(x, T_1^-\right) \\[4mm]
\dfrac{\partial C(x,t)}{\partial x} \Big|_{\partial\Omega} = 0
\end{cases}
$$

where x, *t* represent space and time, respectively, *C* is a concentration of the dissolved organic matters in the target river, $T_l$ is upstream incoming flow start time in the target river, $T_2$ represents incoming flow end time, $\beta$ represents a fractional order capacity coefficient, $\lambda$ represents a truncation coefficient, $\alpha$ represents a fractional order number, *R* represents a reaction coefficient, *v* and *D* are a flow parameter and a diffusion parameter, respectively, *A* represents an initial concentration of the dissolved organic matters, $\partial\Omega$ represents derivative calculation along a boundary, ${}^{TC}_{0}D_t^{\alpha_0,\lambda_0}$ and ${}^{TC}_{T_1}D_t^{\alpha_1,\lambda_1}$ are truncated fractional order derivatives, and the subscripts 0 and 1 of $\alpha, \beta, \lambda, R,$ *v* and *D* represent time periods corresponding to the parameters, respectively, the parameters in the base flow stage being denoted with the subscript 0, and the parameters in the incoming flow stage being denoted with the subscript 1.

According to the method for constructing a transport model for dissolved organic matters in a river provided in embodiments of the present application, the first environmental parameters and the first dissolved organic matter concentrations in the target river in the base flow stage and the incoming flow stage are acquired. The first dissolved organic matter transport mechanism in the target river is determined according to the first environmental parameters and the first dissolved organic matter concentrations, and the dissolved organic matter transport model is constructed, for dissolved organic matters in the target river in the base flow stage and the incoming flow stage, based on the first dissolved organic matter transport mechanism. The dissolved organic matter transport model constructed in the present invention can be used, for the dissolved organic matters in the river under a dynamic condition of a sudden change in an incoming flow, to assist in assessing the influence of the incoming flow, such as rainfall or flood discharge, on the distribution of organic matters in aquatic habitats in the river.

The present invention provides not only the method for constructing a transport model for dissolved organic matters in a river, but also a method for predicting a transport process of dissolved organic matters in a river. Introduced in the above embodiment is mainly a process of constructing a transport model for dissolved organic matters in a river. How to use the constructed dissolved organic matter transport model to predict a transport process of dissolved organic matters in a river will be introduced below. Specifically, in another embodiment of the present invention, a method for predicting a transport process of dissolved organic matters in a river will be introduced. See specifically a schematic flow diagram of a method shown in Fig. 2. Fig. 2 is a schematic flow diagram of a method for predicting a transport process of dissolved organic matters in a target river provided in the present invention. As shown in Fig. 2, the method includes the following steps:

step 210: second environmental parameters and second dissolved organic matter concentrations in a target river in a base flow stage and an incoming flow stage are acquired.

To predict a transport process of dissolved organic matters in a target river in a certain time period, experimental data for the time period is acquired first, i.e., in a prediction stage, the second environmental parameters and the second dissolved organic matter concentrations in the target river in the base flow stage and the incoming flow stage are acquired first. The specific acquiring step is the same as the process of acquiring the first environmental parameters and the first dissolved organic matter concentrations in step 110, and will not be described here.

Step 220: a second dissolved organic matter transport mechanism in the target river is determined according to the second environmental parameters and the second dissolved organic matter concentrations.

The specific step of step 220 is the same as the description in step 120, and will not be described here.

Step 230: the parameters of the dissolved organic matter transport model are calibrated according to the second dissolved organic matter concentrations and the second dissolved organic matter transport mechanism.

Specifically, the calibrated model parameters include a truncation coefficient, a fractional order number, a reaction coefficient, a flow parameter and a diffusion parameter. By using the acquired second dissolved organic matter concentrations, in combination with analysis of the second dissolved organic matter transport mechanism, the parameters of the dissolved organic matter transport model constructed in the method described in any of the above embodiments are calibrated, and fractional order derivative and truncation coefficient ranges in the two stages are compared. The influence of rainfall on the retention characteristics of the dissolved organic matters in the river is analyzed. In general, the lower the fractional order derivative and truncation coefficient, the higher the proportion of a low-velocity region or a deep-water region of the river and the more complex the riverbed structure. Meanwhile, the calibrated reaction coefficient is analyzed, and this parameter reflects the strength of physical and chemical effects on the dissolved organic matters in the transport process. The higher the coefficient, the stronger the physical adsorption and chemical effects.

Step 240: dissolved organic matter distribution are determined based on the dissolved organic matter transport model and the calibrated model parameters.

Specifically, dissolved organic matter distribution is acquired by solving the dissolved organic matter transport model using an implicit finite difference method, based on the dissolved organic matter transport model and the calibrated model parameters.

In the following, solving the dissolved organic matter transport model using an implicit finite difference method is specifically as follows:

when $t \in [0, T_1]$, the number of points distributed in time is $m$, a time step is $\tau = T_1/m$, and a spatial step is $h$, $t_{k+1}$ represents $(k+1)$th time, and $x_l$ represents an $l$th spatial position;

when $k=0$:

$$\left( \tau^{-1} + \beta_0 \frac{\tau^{-\alpha_0}}{\Gamma(2-\alpha_0)} - \frac{2D_0}{h^2} - \beta_0 \lambda_0^{\alpha_0} + R_0 \right) C(x_l, t_1)$$

$$+ \left( \frac{v_0}{h} + \frac{D_0}{h^2} \right) C(x_{l+1}, t_1)$$

$$+ \left( -\frac{v_0}{h} + \frac{D_0}{h^2} \right) C(x_{l-1}, t_1)$$

$$= \tau^{-1} C(x_l, 0) - \left( \beta_0 \frac{e^{-\lambda_0 t_1} \tau^{-\alpha_0}}{\Gamma(2-\alpha_0)} \right) C(x_l, 0)$$

when $k=1$:

$$\left( \tau^{-1} + \beta_0 \frac{\tau^{-\alpha_0}}{\Gamma(2-\alpha_0)} - \frac{2D_0}{h^2} - \beta_0 \lambda_0^{\alpha_0} + R_0 \right) C(x_l, t_2)$$

$$+ \left( \frac{v_0}{h} + \frac{D_0}{h^2} \right) C(x_{l+1}, t_2)$$

$$+ \left( -\frac{v_0}{h} + \frac{D_0}{h^2} \right) C(x_{l-1}, t_2)$$

$$= \tau^{-1} C(x_l, t_1) - \left( \beta_0 \frac{e^{-\lambda_0 t_2} \tau^{-\alpha_0}}{\Gamma(2-\alpha_0)} \right) \left[ \begin{array}{c} e^{\lambda_0 t_1} C(x_l, t_1)(2^{1-\alpha_0} - 2) \\ -C(x_l, 0)(2^{1-\alpha_0} - 1) \end{array} \right]$$

when $k \geq 1$:

$$\left( \tau^{-1} + \beta_0 \frac{\tau^{-\alpha_0}}{\Gamma(2-\alpha_0)} - \frac{2D_0}{h^2} - \beta_0 \lambda_0^{\alpha_0} + R_0 \right) C(x_l, t_{k+1})$$

$$+ \left( \frac{v_0}{h} + \frac{D_0}{h^2} \right) C(x_{l+1}, t_{k+1})$$

$$+ \left( -\frac{v_0}{h} + \frac{D_0}{h^2} \right) C(x_{l-1}, t_{k+1})$$

$$= \tau^{-1} C(x_l, t_k) - \beta_0 \frac{e^{-\lambda_0 t_{k+1}} \tau^{-\alpha_0}}{\Gamma(2-\alpha_0)} e^{\lambda_0 t_k} C(x_l, t_k)(2^{1-\alpha_0} - 2)$$

$$- \beta_0 \frac{e^{-\lambda_0 t_{k+1}} \tau^{-\alpha_0}}{\Gamma(2-\alpha_0)} \sum_{j=1}^{k-1} e^{-\lambda_0 t_{k-j}} C(x_l, t_{k-j}) \left[ (j+2)^{1-\alpha_0} - 2(j+1)^{1-\alpha_0} + j^{1-\alpha_0} \right]$$

$$+ \beta_0 \frac{e^{-\lambda_0 t_{k+1}} \tau^{-\alpha_0}}{\Gamma(2-\alpha_0)} C(x_l, 0) \left[ j_{k+1}^{1-\alpha_0} - j_k^{1-\alpha_0} \right]$$

when $t \in [T_1, T_2]$, the number of points distributed in time is $m_1$, a time step is $\tau_1 = (T_2 - T_1)/m_1$, and a spatial step is still $h$, $t_{k+1}$ represents (k+1)th time, and x, represents an $l$th spatial position;

when $k-m=0$:

$$\left( \tau_1^{-1} + \beta_1 \frac{\tau_1^{-\alpha_1}}{\Gamma(2-\alpha_1)} - \frac{2D_1}{h^2} - \beta_1 \lambda_1^{\alpha_1} + R_1 \right) C(x_l, t_{k+1})$$

$$+ \left( \frac{v_1}{h} + \frac{D_1}{h^2} \right) C(x_{l+1}, t_{k+1})$$

$$+ \left( -\frac{v_1}{h} + \frac{D_1}{h^2} \right) C(x_{l-1}, t_{k+1})$$

$$= \tau_1^{-1} C(x_l, t_k) - \left( \beta_1 \frac{e^{-\lambda_1(t_{k+1}-T_1)} \tau_1^{-\alpha_1}}{\Gamma(2-\alpha_1)} \right) C(x_l, T_1)$$

when *k-m*=1:

$$\left( \tau_1^{-1} + \beta_1 \frac{\tau_1^{-\alpha_1}}{\Gamma(2-\alpha_1)} - \frac{2D_1}{h^2} - \beta_1 \lambda_1^{\alpha_1} + R_1 \right) C(x_l, t_{k+1})$$

$$+ \left( \frac{v_1}{h} + \frac{D_1}{h^2} \right) C(x_{l+1}, t_{k+1})$$

$$+ \left( -\frac{v_1}{h} + \frac{D_1}{h^2} \right) C(x_{l-1}, t_{k+1}) = \tau_1^{-1} C(x_l, t_k)$$

$$- \left( \beta_1 \frac{e^{-\lambda_1(t_{k+1}-T_1)} \tau_1^{-\alpha_1}}{\Gamma(2-\alpha_1)} \right) \left[ \begin{array}{l} e^{\lambda_1(t_k-T_1)} C(x_l, t_k)(2^{1-\alpha_1}-2) \\ -C(x_l, T_1)(2^{1-\alpha_1}-1) \end{array} \right]$$

when *k-m*≥1:

$$\left(\tau_1^{-1}+\beta_1\frac{\tau_1^{-\alpha_1}}{\Gamma(2-\alpha_1)}-\frac{2D_1}{h^2}-\beta_1\lambda_1^{\alpha_1}+R_1\right)C\left(x_l,t_{k+1}\right)$$

$$+\left(\frac{v_1}{h}+\frac{D_1}{h^2}\right)C\left(x_{l+1},t_{k+1}\right)$$

$$+\left(-\frac{v_1}{h}+\frac{D_1}{h^2}\right)C\left(x_{l-1},t_{k+1}\right)$$

$$=\tau_1^{-1}C\left(x_l,t_k\right)-\beta_1\frac{e^{-\lambda_1(t_{k+1}-T_1)}\tau_1^{-\alpha_1}}{\Gamma(2-\alpha_1)}e^{\lambda_1(t_{k+1}-T_1)}C\left(x_l,t_k\right)\left(2^{1-\alpha_1}-2\right)$$

$$-\beta_1\frac{e^{-\lambda_1(t_{k+1}-T_1)}\tau_1^{-\alpha_1}}{\Gamma(2-\alpha_1)}\sum_{j=1}^{k-m-1}e^{-\lambda_1(t_{k-j}-T_1)}C\left(x_l,t_{k-j}\right)\left[(j+2)^{1-\alpha_1}-2(j+1)^{1-\alpha_1}+j^{1-\alpha_1}\right]$$

$$+\beta_1\frac{e^{-\lambda_1(t_{k+1}-T_1)}\tau_1^{-\alpha_1}}{\Gamma(2-\alpha_1)}C\left(x_l,T_1\right)\left[j_{k+1}^{1-\alpha_1}-j_k^{1-\alpha_1}\right]$$

where $x, t$ represent space and time, respectively, $C$ is a concentration of the dissolved organic matters in the target river, $T_l$ is upstream incoming flow start time in the target river, $T_2$ represents incoming flow end time, $\beta$ represents a fractional order capacity coefficient, $\lambda$ represents a truncation coefficient, $\alpha$ represents a fractional order number, $R$ represents a reaction coefficient, $v$ and $D$ are a flow parameter and a diffusion parameter, respectively, $A$ represents an initial concentration of the dissolved organic matters, $\partial\Omega$ represents derivative calculation along a boundary, ${}_{0}^{TC}D_t^{\alpha_0,\lambda_0}$ and ${}_{T_1}^{TC}D_t^{\alpha_1,\lambda_1}$ are truncated fractional order derivatives, the subscripts 0 and 1 of $\alpha$, $\beta$, $\lambda$, $R$, $v$ and $D$ represent time periods corresponding to the parameters, respectively, the parameters in the base flow stage being denoted with the subscript 0, and the parameters in the incoming flow stage being denoted with the subscript 1, $\Gamma$ () is a single-parameter Gamma function, $t_1$ is initial time of a fractional order derivative, $t_2$ is end time of the fractional order derivative, and $e$ is a natural constant.

Step 250: dissolved organic matter distribution are acquired by solving the dissolved organic matter transport model using an implicit finite difference method, based on the dissolved organic matter transport model and the calibrated model parameters.

Based on the dissolved organic matter distribution, a transport process of dissolved organic matters in the region or a similar river channel under a condition of a sudden change in an incoming flow such as rainfall or aerial discharge is predicted, and the influence of this working condition on the distribution of dissolved organic matters in aquatic habitats in the river is assessed. Generally speaking, rainfall or aerial discharge, i.e. a sudden incoming flow, reduces the content of dissolved organic matters upstream, while the content of dissolved organic matters downstream increases.

According to the method for predicting a transport process of dissolved organic matters in a river proposed in the present invention, the second environmental parameters and the second dissolved organic matter concentrations in the target river in the base flow stage and the incoming flow stage are acquired. The second dissolved organic matter transport mechanism in the target river is determined according to the second environmental parameters and the second dissolved organic matter concentrations. The parameters of the dissolved organic matter transport model constructed by the method of any embodiment in the first aspect are calibrated according to the second dissolved organic matter concentrations and the second dissolved organic matter transport mechanism. Dissolved organic matter distribution is determined based on the dissolved organic matter transport model and the calibrated model parameters. A dissolved organic matter transport process in the incoming flow stage in the target river is predicted based on the dissolved organic matter distribution. For dissolved organic matters in the river under a condition of a sudden change in an incoming flow

14

such as rainfall or aerial discharge, a transport process of the dissolved organic matters in the river is effectively simulated, and the distribution of dissolved organic matters in aquatic habitats in the river is dynamically assessed.

A specific embodiment is used below to verify the construction of the dissolved organic matter transport model and the prediction of the dissolved organic matter transport process based on the constructed dissolved organic matter transport model as introduced above in the present invention.

A river selected is Mayfield Creek (32.965453 N, 87.408953 E) in Alabama, which is a second-order coastal plain stream located within the National Forest in Talladega in the southeastern United States, in a subtropical climate zone, with an average annual temperature of 15°C and an average annual precipitation of 672mm. The Mayfield River flows northward into the Black Warrior River and eventually into the Gulf of Mexico. Its watershed soil is moderately well-drained fine sandy loam, and riverbed sediments are mainly sandy particles containing quartz, illite, montmorillonite and kaolinite. For tracer experimental simulation results available, see the document for details:

Song W, Yuehan C, Shuo C, et al. Fractional-derivative model simulations of reach-scale uptake and transport dynamics of natural fluorescent dissolved organic matter in a temperate forested stream in southeastern U.S [J]. Journal of Hydrology, 2021.

In the following, a dissolved organic matter transport process under a condition of a sudden increase of an upstream incoming flow is simulated in conjunction with the technical solution described above in the present invention. The process includes the following steps:

step (1): for the Mayfield Creek in Alabama, a field tracer experiment was conducted and experimental data were collected. For the experimental data, see the document mentioned above for details.

Step (2): based on the experimental data, a transport law of dissolved organic matters in the river was analyzed, the dissolved organic matters containing a variety of functional groups and having large molecular chains and easily adhering to solid surfaces, and thus staying in a low-velocity region of the river for a long time. Therefore, when a peak passes through the low-velocity region, more soluble organic matters are bound to the low-velocity region due to the highest concentration gradient. After the peak passes, a dissolved organic matter concentration in the low-velocity region is higher than that of a flow region, and the bound dissolved organic matters are slowly released, which is expressed as a tailing phenomenon.

Step (3): based on an analysis result in step (2), a dissolved organic matter transport model suitable for the Mayfield Creek in Alabama was established to describe the migration law of the dissolved organic matters.

Specifically, the dissolved organic transport model established is specifically a truncated fractional order derivative model with dual stress sections, which is described in detail previously and will not be repeated here.

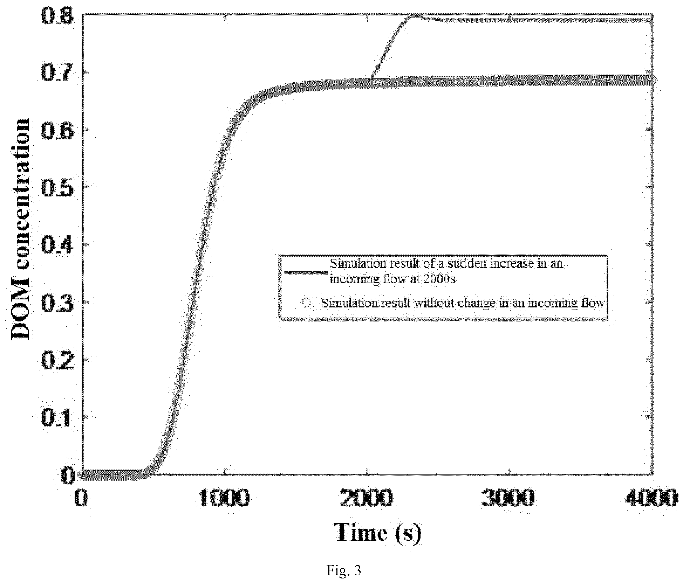

Step (4): model parameters under conditions with and without a change in an incoming flow were calibrated, wherein a fractional order number of the model in a simulation domain is 0.9, and a fractional order capacity coefficient $\beta$ is 0.15; and a working condition was added: a dissolved organic matter concentration at 100m downstream under a condition of a sudden increase in the incoming flow at 2000s. See Fig. 3 for simulation results of the two working conditions. See Table 1 for the model parameters, which are parameters of a control equation for a breakthrough curve in Fig. 3.

Table 1 model parameters

| Curve | $\alpha$ | $\beta$ | D | $\lambda$ | R | v |
|---|---|---|---|---|---|---|
| Without change in an incoming flow | 0.9 | 0.15 | 0.22 | 0.0002 | 0.00055 | 0.15 |
| Sudden increase in the incoming flow at 2000s | [0.9, 0.95] | [0.15, 0.15] | [0.22, 0.22] | 0.0002 | 0.00055 | [0.15, 0.35] |

Step (5): as can be seen from the results in Fig. 3, there is a continuous input of a dissolved organic matter source upstream, and the corresponding concentration of dissolved organic matters downstream increases under the condition of a sudden increase in the incoming flow at 2000s. It indicates that under the condition of a sudden increase in the incoming flow, more dissolved organic matters are rushed to the downstream, which leads to a decrease in the content and concentration of dissolved organic matters upstream. It is speculated that the biodiversity in the downstream correspondingly increases, and the biodiversity in the upstream correspondingly decreases. For toxic dissolved organic matters, the sudden increase in the incoming flow improves the river's ability to clean up pollution.

It can be seen from the above analysis that by predicting the dissolved organic matter transport process using the dissolved organic matter transport model constructed by the method for constructing a transport model for dissolved organic matters provided in the present invention, migration and transformation laws of dissolved organic matters in a river channel influenced by rainfall and other incoming flow changes in the natural environment can be described, and the distribution of dissolved organic matters in aquatic habitats in the river can be assessed dynamically.

Described above are method embodiments of constructing a transport model for dissolved organic matters in a river and predicting a transport process of dissolved organic matters in a river by using the constructed dissolved organic matter transport model, etc. The following is description of embodiments of an apparatus, an electronic device and a storage medium provided in the present application, specifically as follows.

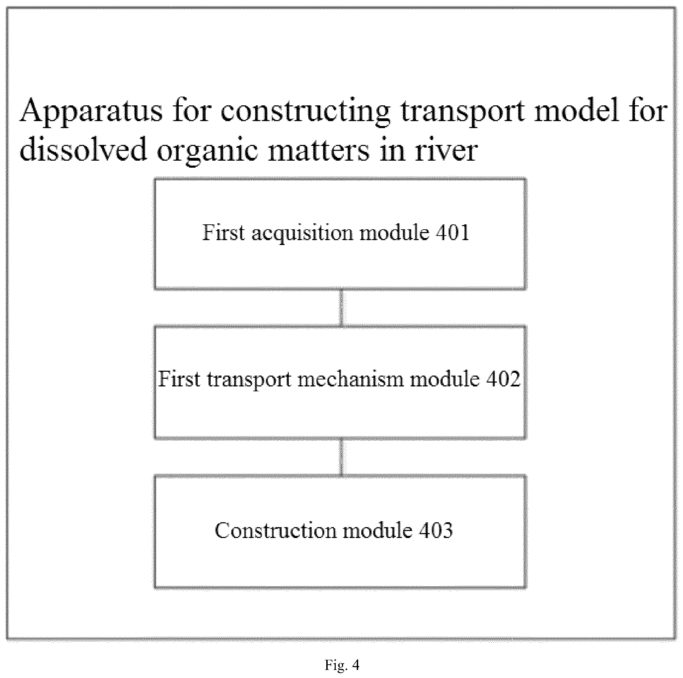

Fig. 4 is a schematic structural diagram of an apparatus for constructing a transport model for dissolved organic matters in a river provided in embodiments of the present invention, as shown in Fig. 4, the apparatus including: a first acquisition module 401, a first transport mechanism module 402, and a construction module 403.

The first acquisition module 401 is configured to acquire first environmental parameters and first dissolved organic matter concentrations in a target river in a base flow stage and an incoming flow stage;

the first transport mechanism module 402 is configured to determine a first dissolved organic matter transport mechanism in the target river according to the first environmental parameters and the first dissolved organic matter concentrations; and

the construction module 403 is configured to construct a dissolved organic matter transport model, for dissolved organic matters in the target river in the base flow stage and the incoming flow stage, based on the dissolved organic matter transport mechanism.

In a possible implementation, acquiring dissolved organic matter distribution based on the dissolved organic matter transport model and the calibrated model parameters includes:
acquiring dissolved organic matter distribution by solving the dissolved organic matter transport model using an implicit finite difference method, based on the dissolved organic matter transport model and the calibrated model parameters.

In an example, the first dissolved organic matter transport mechanism includes:
a physical adsorption ratio biodegradation process of the dissolved organic matters in the target river, and influences, by biochemical and physical effects, on a tailing degree of the dissolved organic matters.

In an example, the dissolved organic matter transport model is:

$$
\begin{cases}
\dfrac{\partial C(x,t)}{\partial t} + \beta_0 \, {}^{TC}_{\ 0}D_t^{\alpha_0,\lambda_0}C(x,t) - \left(\beta_0\lambda_0^{\alpha_0} - R_0\right)C(x,t) \\[2mm]
= -v_0\dfrac{\partial C(x,t)}{\partial x} + D_0\dfrac{\partial^2 C(x,t)}{\partial x^2}, \ t\in\left[0,T_1\right] \\[3mm]
\dfrac{\partial C(x,t)}{\partial t} + \beta_1 \, {}^{TC}_{\ T_1}D_t^{\alpha_1,\lambda_1}C(x,t) - \left(\beta_1\lambda_1^{\alpha_1} - R_1\right)C(x,t) \\[2mm]
= -v_1\dfrac{\partial C(x,t)}{\partial x} + D_1\dfrac{\partial^2 C(x,t)}{\partial x^2}, \ t\in\left[T_1,T_2\right] \\[3mm]
C(x,0) = \begin{cases} A, \ x=0 \\ 0, \ else \end{cases}, \ C\left(x,T_1^+\right) = C\left(x,T_1^-\right) \\[3mm]
\dfrac{\partial C(x,t)}{\partial x}\Big|_{\partial\Omega} = 0
\end{cases}
$$

where x, *t* represent space and time, respectively, *C* is a concentration of the dissolved organic matters in the target

river, $T_1$ is upstream incoming flow start time in the target river, $T_2$ represents incoming flow end time, $\beta$ represents a fractional order capacity coefficient, $\lambda$ represents a truncation coefficient, $\alpha$ represents a fractional order number, $R$ represents a reaction coefficient, $v$ and $D$ are a flow parameter and a diffusion parameter, respectively, $A$ represents an initial concentration of the dissolved organic matters, $\partial\Omega$ represents derivative calculation along a boundary, ${}^{TC}_{0}D_t^{\alpha_0,\lambda_0}$ and ${}^{TC}_{T_1}D_t^{\alpha_1,\lambda_1}$ are truncated fractional order derivatives, and the subscripts 0 and 1 of $\alpha$, $\beta$, $\lambda$, $R$, $v$ and $D$ represent time periods corresponding to the parameters, respectively, the parameters in the base flow stage being denoted with the subscript 0, and the parameters in the incoming flow stage being denoted with the subscript 1.

The functions performed by the components of the apparatus for constructing a transport model for dissolved organic matters in a river provided in embodiments of the present invention have been described in detail in any of the above embodiments of the method for constructing a transport model for dissolved organic matters in a river, and are therefore not described here.

According to the apparatus for constructing a transport model for dissolved organic matters in a river provided in embodiments of the present invention, the first environmental parameters and the first dissolved organic matter concentrations in the target river in the base flow stage and the incoming flow stage are acquired. The first dissolved organic matter transport mechanism in the target river is determined according to the first environmental parameters and the first dissolved organic matter concentrations, and the dissolved organic matter transport model is constructed, for dissolved organic matters in the target river in the base flow stage and the incoming flow stage, based on the first dissolved organic matter transport mechanism. The dissolved organic matter transport model constructed in the present invention can be used, for the dissolved organic matters in the river under a dynamic condition of a sudden change in an incoming flow, to assist in assessing the influence of the incoming flow, such as rainfall or flood discharge, on the distribution of organic matters in aquatic habitats in the river.

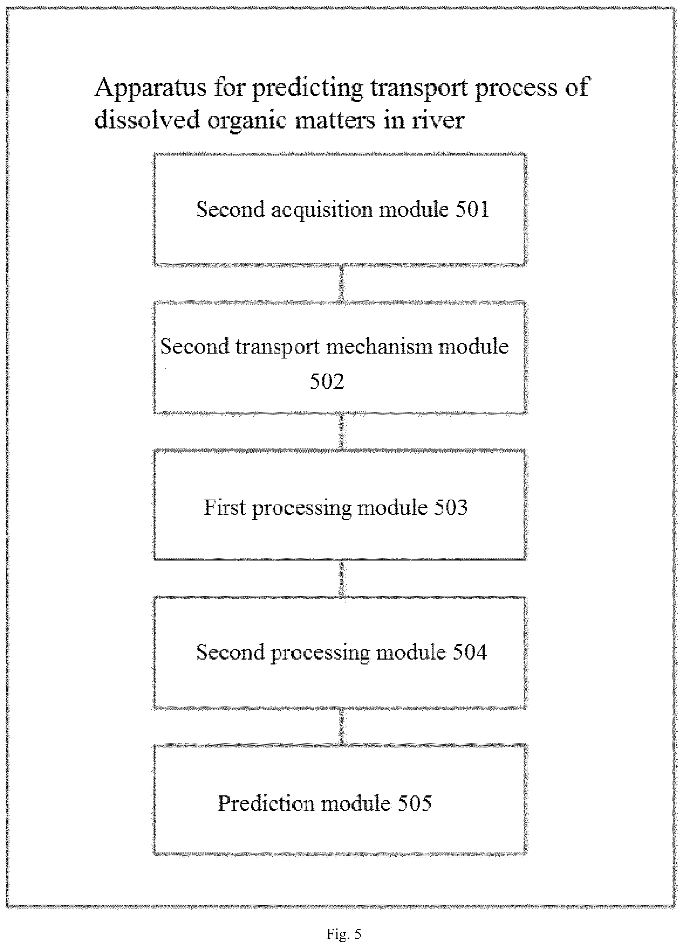

Fig. 5 is a schematic structural diagram of an apparatus for predicting a transport process of dissolved organic matters in a river provided in embodiments of the present invention, as shown in Fig. 5, the apparatus including: a second acquisition module 501, a second transport mechanism module 502, a first processing module 503, a second processing module 504, and a prediction module 505.

The second acquisition module 501 is configured to acquire second environmental parameters and second dissolved organic matter concentrations in a target river in a base flow stage and an incoming flow stage;

the second transport mechanism module 502 is configured to determine a second dissolved organic matter transport mechanism in the target river according to the second environmental parameters and the second dissolved organic matter concentrations;

the first processing module 503 is configured to calibrate the parameters of the dissolved organic matter transport model constructed by the apparatus in the third aspect according to the second dissolved organic matter concentrations and the second dissolved organic matter transport mechanism;

the second processing module 504 is configured to determine dissolved organic matter distribution based on the dissolved organic matter transport model and the calibrated model parameters; and

the prediction module 505 is configured to predict a dissolved organic matter transport process in the incoming flow stage in the target river based on the dissolved organic matter distribution.

In an example, the second processing module 504 is specifically configured to acquire dissolved organic matter distribution by solving the dissolved organic matter transport model using an implicit finite difference method, based on the dissolved organic matter transport model and the calibrated model parameters.

In an example, solving the dissolved organic matter transport model using the implicit finite difference method is specifically as follows:

when $t \in [0, T_1]$, the number of points distributed in time is m, a time step is $\tau = T_1/m$, and a spatial step is $h$, $t_{k+1}$ represents $(k+1)$th time, and $x_l$ represents an $l$th spatial position;

when $k=0$:

$$\left( \tau^{-1} + \beta_0 \frac{\tau^{-\alpha_0}}{\Gamma(2-\alpha_0)} - \frac{2D_0}{h^2} - \beta_0 \lambda_0^{\alpha_0} + R_0 \right) C(x_l, t_1)$$

$$+ \left( \frac{v_0}{h} + \frac{D_0}{h^2} \right) C(x_{l+1}, t_1)$$

$$+ \left( -\frac{v_0}{h} + \frac{D_0}{h^2} \right) C(x_{l-1}, t_1)$$

$$= \tau^{-1} C(x_l, 0) - \left( \beta_0 \frac{e^{-\lambda_0 t_1} \tau^{-\alpha_0}}{\Gamma(2-\alpha_0)} \right) C(x_l, 0)$$

when $k = 1$:

$$\left( \tau^{-1} + \beta_0 \frac{\tau^{-\alpha_0}}{\Gamma(2-\alpha_0)} - \frac{2D_0}{h^2} - \beta_0 \lambda_0^{\alpha_0} + R_0 \right) C(x_l, t_2)$$

$$+ \left( \frac{v_0}{h} + \frac{D_0}{h^2} \right) C(x_{l+1}, t_2)$$

$$+ \left( -\frac{v_0}{h} + \frac{D_0}{h^2} \right) C(x_{l-1}, t_2)$$

$$= \tau^{-1} C(x_l, t_1) - \left( \beta_0 \frac{e^{-\lambda_0 t_2} \tau^{-\alpha_0}}{\Gamma(2-\alpha_0)} \right) \left[ \begin{array}{c} e^{\lambda_0 t_1} C(x_l, t_1) \left( 2^{1-\alpha_0} - 2 \right) \\ -C(x_l, 0) \left( 2^{1-\alpha_0} - 1 \right) \end{array} \right]$$

when $k \geq 1$:

$$\left(\tau^{-1}+\beta_0\frac{\tau^{-\alpha_0}}{\Gamma(2-\alpha_0)}-\frac{2D_0}{h^2}-\beta_0\lambda_0^{\alpha_0}+R_0\right)C(x_l,t_{k+1})$$

$$+\left(\frac{v_0}{h}+\frac{D_0}{h^2}\right)C(x_{l+1},t_{k+1})$$

$$+\left(-\frac{v_0}{h}+\frac{D_0}{h^2}\right)C(x_{l-1},t_{k+1})$$

$$=\tau^{-1}C(x_l,t_k)-\beta_0\frac{e^{-\lambda_0 t_{k+1}}\tau^{-\alpha_0}}{\Gamma(2-\alpha_0)}e^{\lambda_0 t_k}C(x_l,t_k)\left(2^{1-\alpha_0}-2\right)$$

$$-\beta_0\frac{e^{-\lambda_0 t_{k+1}}\tau^{-\alpha_0}}{\Gamma(2-\alpha_0)}\sum_{j=1}^{k-1}e^{-\lambda_0 t_{k-j}}C(x_l,t_{k-j})\left[(j+2)^{1-\alpha_0}-2(j+1)^{1-\alpha_0}+j^{1-\alpha_0}\right]$$

$$+\beta_0\frac{e^{-\lambda_0 t_{k+1}}\tau^{-\alpha_0}}{\Gamma(2-\alpha_0)}C(x_l,0)\left[{j_{k+1}}^{1-\alpha_0}-{j_k}^{1-\alpha_0}\right]$$

when $t \in [T_1, T_2]$, the number of points distributed in time is $m_1$, a time step is $\tau_1 = (T_2 - T_1)/m_1$, and a spatial step is still $h$, $t_{k+1}$ represents $(k+1)$th time, and $x_l$ represents an $l$th spatial position;

when $k$-$m$=0:

$$\left(\tau_1^{-1}+\beta_1\frac{\tau_1^{-\alpha_1}}{\Gamma(2-\alpha_1)}-\frac{2D_1}{h^2}-\beta_1\lambda_1^{\alpha_1}+R_1\right)C(x_l,t_{k+1})$$

$$+\left(\frac{v_1}{h}+\frac{D_1}{h^2}\right)C(x_{l+1},t_{k+1})$$

$$+\left(-\frac{v_1}{h}+\frac{D_1}{h^2}\right)C(x_{l-1},t_{k+1})$$

$$=\tau_1^{-1}C(x_l,t_k)-\left(\beta_1\frac{e^{-\lambda_1(t_{k+1}-T_1)}\tau_1^{-\alpha_1}}{\Gamma(2-\alpha_1)}\right)C(x_l,T_1)$$

when $k$-$m$=1:

$$\left( \tau_1^{-1} + \beta_1 \frac{\tau_1^{-\alpha_1}}{\Gamma(2-\alpha_1)} - \frac{2D_1}{h^2} - \beta_1 \lambda_1^{\alpha_1} + R_1 \right) C\left(x_l, t_{k+1}\right)$$

$$+ \left( \frac{v_1}{h} + \frac{D_1}{h^2} \right) C\left(x_{l+1}, t_{k+1}\right)$$

$$+ \left( -\frac{v_1}{h} + \frac{D_1}{h^2} \right) C\left(x_{l-1}, t_{k+1}\right) = \tau_1^{-1} C\left(x_l, t_k\right)$$

$$- \left( \beta_1 \frac{e^{-\lambda_1(t_{k+1}-T_1)} \tau_1^{-\alpha_1}}{\Gamma(2-\alpha_1)} \right) \left[ \begin{array}{l} e^{\lambda_1(t_k-T_1)} C\left(x_l, t_k\right)\left(2^{1-\alpha_1} - 2\right) \\ -C\left(x_l, T_1\right)\left(2^{1-\alpha_1} - 1\right) \end{array} \right]$$

when $k-m \geq 1$:

$$\left( \tau_1^{-1} + \beta_1 \frac{\tau_1^{-\alpha_1}}{\Gamma(2-\alpha_1)} - \frac{2D_1}{h^2} - \beta_1 \lambda_1^{\alpha_1} + R_1 \right) C\left(x_l, t_{k+1}\right)$$

$$+ \left( \frac{v_1}{h} + \frac{D_1}{h^2} \right) C\left(x_{l+1}, t_{k+1}\right)$$

$$+ \left( -\frac{v_1}{h} + \frac{D_1}{h^2} \right) C\left(x_{l-1}, t_{k+1}\right)$$

$$= \tau_1^{-1} C\left(x_l, t_k\right) - \beta_1 \frac{e^{-\lambda_1(t_{k+1}-T_1)} \tau_1^{-\alpha_1}}{\Gamma(2-\alpha_1)} e^{\lambda_1(t_{k+1}-T_1)} C\left(x_l, t_k\right)\left(2^{1-\alpha_1} - 2\right)$$

$$- \beta_1 \frac{e^{-\lambda_1(t_{k+1}-T_1)} \tau_1^{-\alpha_1}}{\Gamma(2-\alpha_1)} \sum_{j=1}^{k-m-1} e^{-\lambda_1(t_{k-j}-T_1)} C\left(x_l, t_{k-j}\right)\left[ (j+2)^{1-\alpha_1} - 2(j+1)^{1-\alpha_1} + j^{1-\alpha_1} \right]$$

$$+ \beta_1 \frac{e^{-\lambda_1(t_{k+1}-T_1)} \tau_1^{-\alpha_1}}{\Gamma(2-\alpha_1)} C\left(x_l, T_1\right)\left[ j_{k+1}^{1-\alpha_1} - j_k^{1-\alpha_1} \right]$$

where $x, t$ represent space and time, respectively, $C$ is a concentration of the dissolved organic matters in the target river, $T_1$ is upstream incoming flow start time in the target river, $T_2$ represents incoming flow end time, $\beta$ represents a fractional order capacity coefficient, $\lambda$ represents a truncation coefficient, $\alpha$ represents a fractional order number, $R$ represents a reaction coefficient, $v$ and $D$ are a flow parameter and a diffusion parameter, respectively, $A$ represents an initial concentration of the dissolved organic matters, $\partial\Omega$ represents derivative calculation along a boundary,

$^{TC}_{0}D^{\alpha_0,\lambda_0}_{t}$ and $^{TC}_{T_1}D^{\alpha_1,\lambda_1}_{t}$ are truncated fractional order derivatives, the subscripts 0 and 1 of $\alpha$, $\beta$, $\lambda$, *R*, *v* and *D* represent time periods corresponding to the parameters, respectively, the parameters in the base flow stage being denoted with the subscript 0, and the parameters in the incoming flow stage being denoted with the subscript 1, $\Gamma$ () is a single-parameter Gamma function, $t_1$ is initial time of a fractional order derivative, $t_2$ is end time of the fractional order derivative, and *e* is a natural constant.

The functions performed by the components of the apparatus for predicting a transport process of dissolved organic matters in a river provided in embodiments of the present invention have been described in detail in any of the above embodiments of the method for predicting a transport process of dissolved organic matters in a river, and are therefore not described here.

According to the apparatus for predicting a transport process of dissolved organic matters in a river provided in embodiments of the present invention, the second environmental parameters and the second dissolved organic matter concentrations in the target river in the base flow stage and the incoming flow stage are acquired. The second dissolved organic matter transport mechanism in the target river is determined according to the second environmental parameters and the second dissolved organic matter concentrations. The parameters of the dissolved organic matter transport model constructed by the method of any embodiment in the first aspect are calibrated according to the second dissolved organic matter concentrations and the second dissolved organic matter transport mechanism. Dissolved organic matter distribution is determined based on the dissolved organic matter transport model and the calibrated model parameters. A dissolved organic matter transport process in the incoming flow stage in the target river is predicted based on the dissolved organic matter distribution. For dissolved organic matters in the river under a condition of a sudden change in an incoming flow such as rainfall or aerial discharge, a transport process of the dissolved organic matters in the river is effectively simulated, and the distribution of dissolved organic matters in aquatic habitats in the river is dynamically assessed.

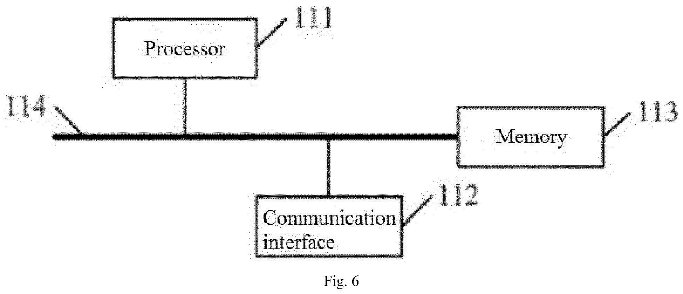

Fig. 6 is a schematic structural diagram of an electronic device provided in embodiments of the present invention. A shown in Fig. 6. embodiments of the present application provide an electronic device, including a processor 111, a communication interface 112, a memory 113 and a communication bus 114, wherein the processor 111, the communication interface 112 and the memory 113 communicate with each other via the communication bus 114.

The memory 113 is configured to store a computer program; and

in one embodiment of the present application, the processor 111 is configured to implement the steps of the method for constructing a transport model for dissolved organic matters in a river provided in any of the above method embodiments, when executing the program stored in the memory 113.

Or the processor 111 is configured to implement the steps of the method for predicting a transport process of dissolved organic matters in a river provided in any of the above method embodiments, when executing the program stored in the memory 113.

Embodiments of the present application also provide a computer readable storage medium, with a computer program stored therein, wherein the computer program, when executed by a processor, implements the steps of the method for constructing a transport model for dissolved organic matters in a river provided in any of the above method embodiments;

or the computer program, when executed by a processor, implements the steps of the method for predicting a transport process of dissolved organic matters in a river provided in any of the above method embodiments.

Professionals should also realize that the units and algorithmic steps of the examples described in conjunction with the embodiments disclosed herein can be implemented by means of electronic hardware, computer software, or a combination of both. To clearly illustrate the interchangeability of hardware and software, the composition and steps of each example have been described generally by functions in the above description. Whether these functions are performed in hardware or software depends on the specific application and design constraints of the technical solution. Professional technical persons may use different methods for each specific application to implement the described functions, but such implementation should not be considered as beyond the scope of the present invention.

The steps of the methods or algorithms described in conjunction with the embodiments disclosed herein may be implemented by means of hardware, a software module executed by a processor, or a combination of both. The software module may be stored in a random access memory (RAM), a memory, a read-only memory (ROM), an electrically programmable ROM, an electrically erasable programmable ROM, a register, a hard disk, a removable disk, a CD-ROM, or a storage medium in any other form known in the technical field.

Described above are only specific embodiments of the present invention to enable those skilled in the art to understand or implement the present invention. Various modifications to these embodiments will be apparent to those skilled in the art, and the general principles defined herein can be implemented in other embodiments without departing from the spirit or scope of the present invention. Therefore, the present invention will not be limited to these embodiments illustrated herein, but will be subject to the widest scope consistent with the principles and novel features applied herein.

**Claims**

1. A method for constructing a transport model for dissolved organic matters in a river, **characterized by** comprising:

acquiring first environmental parameters and first dissolved organic matter concentrations in a target river in a base flow stage and an incoming flow stage;
determining a first dissolved organic matter transport mechanism in the target river according to the first environmental parameters and the first dissolved organic matter concentrations; and
constructing a dissolved organic matter transport model, for dissolved organic matters in the target river in the base flow stage and the incoming flow stage, based on the first dissolved organic matter transport mechanism, wherein the first dissolved organic matter transport mechanism comprises:

a physical adsorption ratio biodegradation process of the dissolved organic matters in the target river, and influences, by biochemical and physical effects, on a tailing degree of the dissolved organic matters; and the dissolved organic matter transport model is:

$$
\begin{cases}
\dfrac{\partial C(x,t)}{\partial t} + \beta_0 \, {}^{TC}_{0}D_t^{\alpha_0,\lambda_0} C(x,t) - \left(\beta_0 \lambda_0^{\alpha_0} - R_0\right) C(x,t) \\[2mm]
= -v_0 \dfrac{\partial C(x,t)}{\partial x} + D_0 \dfrac{\partial^2 C(x,t)}{\partial x^2}, \; t \in \left[0, T_1\right] \\[4mm]
\dfrac{\partial C(x,t)}{\partial t} + \beta_1 \, {}^{TC}_{T_1}D_t^{\alpha_1,\lambda_1} C(x,t) - \left(\beta_1 \lambda_1^{\alpha_1} - R_1\right) C(x,t) \\[2mm]
= -v_1 \dfrac{\partial C(x,t)}{\partial x} + D_1 \dfrac{\partial^2 C(x,t)}{\partial x^2}, \; t \in \left[T_1, T_2\right] \\[4mm]
C(x,0) = \begin{cases} A, \; x = 0 \\ 0, \; else \end{cases}, \; C\left(x, T_1^+\right) = C\left(x, T_1^-\right) \\[4mm]
\dfrac{\partial C(x,t)}{\partial x} \Big|_{\partial \Omega} = 0
\end{cases}
$$

where x, t represent space and time, respectively, C is a concentration of the dissolved organic matters in the target river, $T_1$ is upstream incoming flow start time in the target river, $T_2$ represents incoming flow end time point, $\beta$ represents a fractional order capacity coefficient, $\lambda$ represents a truncation coefficient, $\alpha$ represents a fractional order number, R represents a reaction coefficient, v and D are a flow parameter and a diffusion parameter, respectively, A represents an initial concentration of the dissolved organic matters, $\partial \Omega$ represents derivative calculation along a boundary, ${}^{TC}_{0}D_t^{\alpha_0,\lambda_0}$ and ${}^{TC}_{T_1}D_t^{\alpha_1,\lambda_1}$ are truncated fractional order derivatives, and the subscripts 0 and 1 of $\alpha, \beta, \lambda, R, v$ and D represent time periods corresponding to the parameters, respectively, the parameters in the base flow stage being denoted with the subscript 0, and the parameters in the incoming flow stage being denoted with the subscript 1.

2. A method for predicting a transport process of dissolved organic matters in a river, **characterized by** comprising:

acquiring second environmental parameters and second dissolved organic matter concentrations in a target river in a base flow stage and an incoming flow stage;

determining a second dissolved organic matter transport mechanism in the target river according to the second environmental parameters and the second dissolved organic matter concentrations;

calibrating the parameters of the dissolved organic matter transport model constructed by the method of claim 1 according to the second dissolved organic matter concentrations and the second dissolved organic matter transport mechanism;

determining dissolved organic matter distribution based on the dissolved organic matter transport model and the calibrated model parameters; and

predicting a dissolved organic matter transport process in the incoming flow stage in the target river based on the dissolved organic matter distribution.

3. The method according to claim 2, **characterized in that** acquiring dissolved organic matter distribution based on the dissolved organic matter transport model and the calibrated model parameters comprises:

acquiring dissolved organic matter distribution by solving the dissolved organic matter transport model using an implicit finite difference method, based on the dissolved organic matter transport model and the calibrated model parameters.

4. An apparatus for constructing a transport model for dissolved organic matters in a river, **characterized by** comprising:

a first acquisition module configured to acquire first environmental parameters and first dissolved organic matter concentrations in a target river in a base flow stage and an incoming flow stage;

a first transport mechanism module configured to determine a first dissolved organic matter transport mechanism in the target river according to the first environmental parameters and the first dissolved organic matter concentrations; and

a construction module configured to construct a dissolved organic matter transport model, for dissolved organic matters in the target river in the base flow stage and the incoming flow stage, based on the dissolved organic matter transport mechanism,

wherein the first dissolved organic matter transport mechanism comprises:

a physical adsorption ratio biodegradation process of the dissolved organic matters in the target river, and influences, by biochemical and physical effects, on a tailing degree of the dissolved organic matters; and the dissolved organic matter transport model is:

$$\begin{cases} \dfrac{\partial C(x,t)}{\partial t} + \beta_0 \, {}_{0}^{TC}D_t^{\alpha_0,\lambda_0} C(x,t) - \left( \beta_0 \lambda_0^{\alpha_0} - R_0 \right) C(x,t) \\[2mm] = -v_0 \dfrac{\partial C(x,t)}{\partial x} + D_0 \dfrac{\partial^2 C(x,t)}{\partial x^2}, \ t \in \left[ 0, T_1 \right] \\[3mm] \dfrac{\partial C(x,t)}{\partial t} + \beta_1 \, {}_{T_1}^{TC}D_t^{\alpha_1,\lambda_1} C(x,t) - \left( \beta_1 \lambda_1^{\alpha_1} - R_1 \right) C(x,t) \\[2mm] = -v_1 \dfrac{\partial C(x,t)}{\partial x} + D_1 \dfrac{\partial^2 C(x,t)}{\partial x^2}, \ t \in \left[ T_1, T_2 \right] \\[3mm] C(x,0) = \begin{cases} A, \ x = 0 \\ 0, \ else \end{cases}, \ C\left( x, T_1^+ \right) = C\left( x, T_1^- \right) \\[3mm] \dfrac{\partial C(x,t)}{\partial x} \Big|_{\partial \Omega} = 0 \end{cases}$$

where *x, t* represent space and time, respectively, *C* is a concentration of the dissolved organic matters in the target river, $T_1$ is upstream incoming flow start time in the target river, $T_2$ represents incoming flow end time, $\beta$ represents a fractional order capacity coefficient, $\lambda$ represents a truncation coefficient, $\alpha$ represents a fractional order number, *R* represents a reaction coefficient, *v* and *D* are a flow parameter and a diffusion parameter, respectively, *A* represents an initial concentration of the dissolved organic matters, $\partial\Omega$ represents derivative calculation along a boundary, ${}_{0}^{TC}D_{t}^{\alpha_0,\lambda_0}$ and ${}_{T_1}^{TC}D_{t}^{\alpha_1,\lambda_1}$ are truncated fractional order derivatives, and the subscripts 0 and 1 of $\alpha$, $\beta$, $\lambda$, *R*, *v* and *D* represent time periods corresponding to the parameters, respectively, the parameters in the base flow stage being denoted with the subscript 0, and the parameters in the incoming flow stage being denoted with the subscript 1.

5. An apparatus for predicting a transport process of dissolved organic matters in a river, **characterized by** comprising:

   a second acquisition module configured to acquire second environmental parameters and second dissolved organic matter concentrations in a target river in a base flow stage and an incoming flow stage;
   a second transport mechanism module configured to determine a second dissolved organic matter transport mechanism in the target river according to the second environmental parameters and the second dissolved organic matter concentrations;
   a first processing module configured to calibrate the parameters of the dissolved organic matter transport model constructed by the apparatus of claim 4 according to the second dissolved organic matter concentrations and the second dissolved organic matter transport mechanism;
   a second processing module configured to determine dissolved organic matter distribution based on the dissolved organic matter transport model and the calibrated model parameters; and
   a prediction module configured to predict a dissolved organic matter transport process in the incoming flow stage in the target river based on the dissolved organic matter distribution.

6. An electronic device, comprising a processor, a communication interface, a memory and a communication bus, **characterized in that** the processor, the communication interface and the memory communicate with each other via the communication bus;

   the memory is configured to store a computer program; and
   the processor is configured to implement the steps of the method for constructing a transport model for dissolved organic matters in a river of claim 1 when executing the program stored in the memory;
   or the processor is configured to implement the steps of the method for predicting a transport process of dissolved organic matters in a river of claim 2 or 3 when executing the program stored in the memory.

7. A computer readable storage medium, with a computer program stored therein, **characterized in that** the computer program, when executed by a processor, implements the steps of the method for constructing a transport model for dissolved organic matters in a river of claim 1;
   or, the computer program, when executed by a processor, implements the steps of the method for predicting a transport process of dissolved organic matters in a river of claim 2 or 3.

Acquiring first environmental parameters and first dissolved organic matter concentrations in a target river in a base flow stage and an incoming flow stage  ⌐110

Determining a first dissolved organic matter transport mechanism in the target river according to the first environmental parameters and the first dissolved organic matter concentrations  ⌐120

Constructing a dissolved organic matter transport model, for dissolved organic matters in the target river in the base flow stage and the incoming flow stage, based on the first dissolved organic matter transport mechanism  ⌐130

Fig. 1

Acquiring second environmental parameters and second dissolved organic matter concentrations in a target river in a base flow stage and an incoming flow stage  ⌐210

Determining a second dissolved organic matter transport mechanism in the target river according to the second environmental parameters and the second dissolved organic matter concentrations  ⌐220

Calibrating the parameters of the dissolved organic matter transport model constructed by the method in any embodiment in the first aspect according to the second dissolved organic matter concentrations and the second dissolved organic matter transport mechanism  ⌐230

Determining dissolved organic matter distribution based on the dissolved organic matter transport model and the calibrated model parameters  ⌐240

Predicting a dissolved organic matter transport process in the incoming flow stage in the target river based on the dissolved organic matter distribution  ⌐250

Fig. 2

Fig. 3

Apparatus for constructing transport model for dissolved organic matters in river

First acquisition module 401

First transport mechanism module 402

Construction module 403

Fig. 4

## Apparatus for predicting transport process of dissolved organic matters in river

Second acquisition module 501

Second transport mechanism module 502

First processing module 503

Second processing module 504

Prediction module 505

Fig. 5

Processor 111

114

Memory 113

Communication interface 112

Fig. 6

EP 4 303 879 A1

Europäisches
Patentamt

European
Patent Office

Office européen
des brevets

# EUROPEAN SEARCH REPORT

Application Number

EP 22 20 6560

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WEI SONG ET AL: "Fractional-derivative model simulations of reach-scale uptake and transport dynamics of natural fluorescent dissolved organic matter in a temperate forested stream in southeastern U.S", JOURNAL OF HYDROLOGY, ELSEVIER, AMSTERDAM, NL, vol. 603, 28 August 2021 (2021-08-28), XP086860284, ISSN: 0022-1694, DOI: 10.1016/J.JHYDROL.2021.126878 [retrieved on 2021-08-28] * the whole document * ----- | 1-7 | INV. G16C99/00 |
| | | | TECHNICAL FIELDS SEARCHED (IPC) |
| | | | G16C |

The present search report has been drawn up for all claims

| Place of search | Date of completion of the search | Examiner |
|---|---|---|
| The Hague | 3 August 2023 | Nurmi, Jussi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SONG W ; YUEHAN C ; SHUO C et al.** Fractional-derivative model simulations of reach-scale uptake and transport dynamics of natural fluorescent dissolved organic matter in a temperate forested stream in southeastern U.S [J. *Journal of Hydrology,* 2021